# EUROPEAN PATENT SPECIFICATION

(11) **EP 3 908 364 B1**
(45) Date of publication and mention of the grant of the patent: **24.07.2024**
(21) Application number: 19816757.9
(22) Date of filing: 12.12.2019
(51) Int. Cl.: A61N 1/362

(54) **IMPLANTABLE SYSTEM FOR STIMULATING A HUMAN OR AN ANIMAL HEART**
IMPLANTIERBARE ANORDNUNG ZUR STIMULATION EINES MENSCHLICHEN ODER TIERISCHEN HERZENS
DISPOSITIF POUVANT ÊTRE IMPLANTÉ DESTINÉ À STIMULER UN COEUR HUMAIN OU ANIMAL

(30) Priority: 11.01.2019 DE 102019100610; 02.05.2019 EP 19172205
(43) Date of publication of application: 17.11.2021
(73) Proprietor: BIOTRONIK SE & Co. KG, 12359 Berlin (DE)
(72) Inventor: DOERR, Thomas, 12437 Berlin (DE); LANG, Volker, 12161 Berlin (DE)
(74) Representative: Biotronik Corporate Services SE
(86) International application number: PCT/EP2019/084852
(87) International publication number: WO 2020/143986

(56) References cited:
- US-A1- 2002 120 318
- US-A1- 2012 239 106
- US-B2- 9 168 382

## Description

The present invention relates to an implantable system for stimulating a human or an animal heart according to the preamble of claim 1 and to a computer program product according to the preamble of claim 11.

Implantable systems for stimulating a human or an animal heart, such as cardiac pacemakers, have been known for quite some time. These can carry out a variety of functions. Different stimulation programs may be carried out by a corresponding cardiac pacemaker in the process so as to return the treated heart to a normal state.

A cardiac pacemaker is known from US 8,565,880 B2, which is suitable for stimulating the His bundle. The His bundle is a bundle of specific heart muscle cells that forms part of the cardiac excitation conduction system. The His bundle is located distal from the atrioventricular node, toward the cardiac apex. Since the bundle of His represents a comparatively small unit inside a human or an animal heart, it is difficult at times to be able to correctly contact and excite the His bundle by way of an electrode. The aforementioned US patent relates to an approach for verifying a correct capture of the His bundle and/or dividing the manner of the capture of the His bundle into different categories. It is then possible to predefine different excitation configurations of the cardiac pacemaker as a function of the particular category of the His bundle capture.

US patent US 8,761,880 B2 also relates to the verification of a His bundle capture. A time is determined for this purpose, within which a first piece of cardiac activity information is obtained in response to a His bundle stimulation. The quality of the His bundle capture is ascertained as a function of the verified time interval.

US patent US 9,168,382 B2 likewise relates to methods for determining a His bundle capture. For this purpose, cardiac activity signals are detected, which are generated as a result of an assumed His bundle stimulation. The detected signals are analyzed as to whether these originate from conductive tissue or from myocardial tissue. Only the signals that originate from conductive tissue are used for the selective verification of the His bundle capture.

US patent application US 2002/0120318 A1 discloses a cardiac stimulation device, a His Bundle lead, and an associated method for detecting a conduction signal from the His Bundle. Upon this detection, the stimulation device delivers ventricular stimulation to the right and/or left ventricles at an interval following the atrial depolarization. A sensing window is initiated following the sensing of the atrial depolarization.

US patent application US 2012/0239106 A1 describes methods and systems for providing stimulation energy to a His-bundle to activate natural cardiac contraction mechanisms. It is described that interval information can be used to describe a cardiac response to His-bundle stimulation, and the interval information can provide cardiac stimulation diagnostic information. It is taught that interval information can be used to discriminate between intrinsic conduction cardiac contractions and contractions responsive to His-bundle pacing.

The prior art thus already describes several methods that can be used to verify a correct His bundle capture. However, conventional cardiac pacemakers and implantable defibrillators are typically used for His bundle pacing. These, however, are not adapted to the signal and timer conditions of His bundle pacing. In particular, these conventional systems do not typically allow His bundle signals to be detected with the necessary accuracy. In addition, the conventional systems typically do not provide any specific stimulation modes that are adapted to the requirements of His bundle pacing. Moreover, with the conventional systems, His bundle pacing typically necessitates a restriction of existing safety functions and algorithms, so that these are no longer effectively available to ensure the safety of the conventional systems.

It is the object of the present invention to overcome the drawbacks of conventional cardiac pacemakers with respect to a His bundle stimulation known from the prior art, and to provide a device that is particularly well-suited for His bundle stimulation.

This object is achieved by an implantable system for stimulating a human heart or an animal heart having the features of claim 1. Such a system comprises a first stimulation unit and a first detection unit. The first stimulation unit is used to stimulate at least one ventricle of a human heart or of an animal heart. The first detection unit is used to detect an electrical signal of at least one ventricle of the same human or animal heart. This may, for example, be an electrical signal that was generated as a result of a prior stimulation by the stimulation unit. However, this may also be an electrical signal of the ventricle that was generated intrinsically (without prior external stimulation) by the ventricle.

According to the invention, it is provided that the implantable system comprises a second stimulation unit, which is specifically designed and configured to stimulate the His bundle of the same human or animal heart. This means that a stimulation of the His bundle of the heart does not take place by way of the conventionally used stimulation unit, but by way of a separate stimulation unit, which is likewise provided in the system. The system comprises a first timer, which is used to deliver stimulation pulses by the first stimulation unit in a chronologically defined manner. In addition, the system, in this variant, comprises a second timer, which is provided and configured to match the delivery point in time at which at least one pulse to be delivered by the second stimulation unit to a delivery point in time of at least one pulse to be delivered by the first stimulation unit. Whereas the implantable systems for stimulating the human or animal heart known from the prior art frequently comprise a single timer, which can be used to carry out a stimulation therapy in a chronological sequence that is meaningful for the heart to be stimulated, the provision of a second timer, which ensures synchronization between the second stimulation unit and the first stimulation unit, is not known from the prior art. This second timer thus allows the stimulation of the His bundle to be synchronized with the stimulation of another cardiac region by the first stimulation unit. In this way, a synergistic effect can be achieved particularly easily between the His bundle stimulation and conventional stimulation of another cardiac region.

In one variant, the second timer is not only used to synchronize a His bundle stimulation with a conventional stimulation of another cardiac region, but also to synchronize such a conventional stimulation of a cardiac region with a His bundle stimulation to be carried out by the same implantable system. It is provided in this variant, for example, that the second timer is also used to match the delivery point in time of at least one pulse that is to be delivered by the first stimulation unit to a point in time at which at least one pulse is to be delivered by the second stimulation unit.

According to an embodiment, the second stimulation unit is optimized with respect to the requirements that exist on the part of the His bundle in terms of pacing. In detail, the second stimulation unit is thus specifically designed for His bundle stimulation and configured to have a maximum stimulation energy that is at least 10% higher than the maximum stimulation energy of the first stimulation unit. As a result of this higher maximum stimulation energy, better stimulation of the His bundle can take place. The reason is that, compared to other regions of the heart, the His bundle is comparatively difficult to excite, thereby typically requiring more energy. In contrast, such a higher stimulation energy is not necessary or provided with conventional cardiac pacemakers. These are, in general, only used to stimulate those cardiac regions that can already be easily stimulated using a lower stimulation energy.

Further details of aspects of the invention are described in greater detail hereafter in connection with exemplary embodiments and drawings. In the drawings:
- FIG. 1: shows a block diagram of an exemplary embodiment of an implantable system for stimulating the human heart or animal heart;
- FIG. 2: shows a schematic representation of an exemplary embodiment of an implantable system for stimulation of the human or animal heart;
- FIG. 3: shows a block diagram of an exemplary embodiment of an implantable system for stimulating the human or animal heart, which carries out stimulation outcome monitoring during operation;
- FIG. 4: shows a simplified block diagram of an exemplary embodiment of an analysis device;
- FIG. 5: shows an exemplary embodiment of a representation output by the analysis device from FIG. 4; and
- FIG. 6: shows a block diagram of an exemplary embodiment of an implantable system for stimulating the human or animal heart.

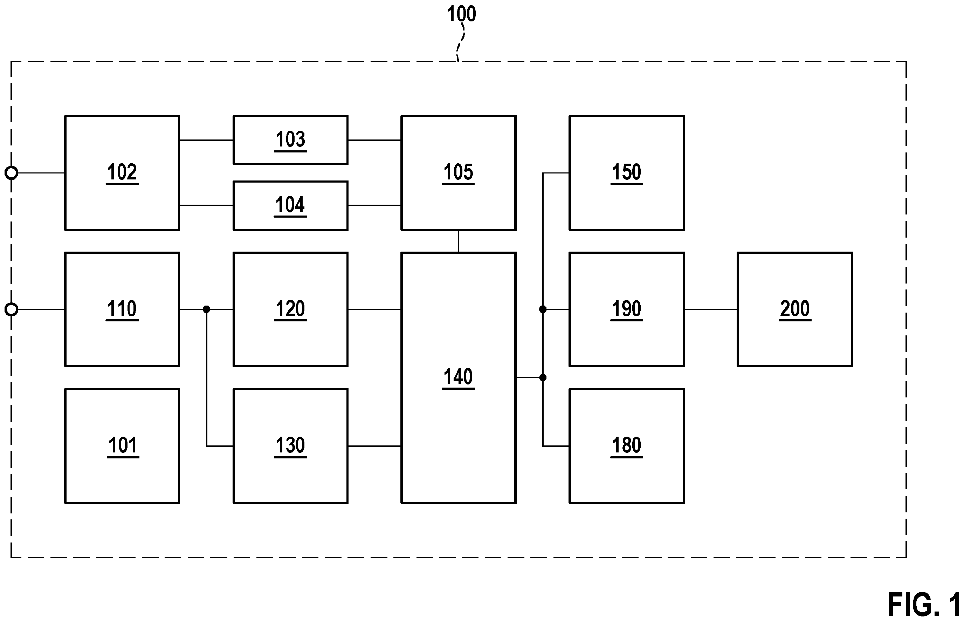

FIG. 1 shows a block diagram of an exemplary embodiment of a cardiac pacemaker 100, which is used as an implantable system for stimulating the human or animal heart. The cardiac pacemaker 100 comprises a power source 101 and a first electrode terminal 102, which serves as a first stimulation output. A first stimulation unit 103, from which stimulation pulses can be conducted through the first electrode terminal 102 to a first electrode, is connected to the electrode terminal 102. Moreover, a first detection unit 104 is connected to the first electrode terminal 102. The first stimulation unit 103 and the first detection unit 104 act as conventional detection and stimulation stages of the cardiac pacemaker 100.

So as to ensure a defined delivery of stimulation pulses, in terms of time, by the first stimulation unit 103 and to adapt the corresponding stimulation pulses to the signals of a heart detected by the first detection unit 104, the cardiac pacemaker 100 further comprises a first timer 105, which likewise functions in a conventional manner.

In addition, the cardiac pacemaker 100 comprises a second electrode terminal 110, which serves as a second stimulation output. It is connected to a second stimulation unit 120 and a second detection unit 130. The second stimulation unit is specifically designed and configured to carry out a stimulation of the His bundle of the heart to be treated. The second detection unit 130 is specifically designed and configured to detect an electrical signal of the His bundle of this heart.

Both the second stimulation unit 120 and the second detection unit 130 are connected to a second timer 140. The timer is additionally operatively connected to the first timer 105. It is possible, by way of the timer 140, to synchronize the stimulation pulses to be delivered by the second stimulation unit 120 with the stimulation pulses being delivered by the first stimulation unit 103. In this way, the His bundle of the heart can be stimulated at a point in time at which the His bundle is particularly receptive to such a stimulation and which is an obvious choice, in a physiologically meaningful manner, for restoring a natural cardiac rhythm. The second timer 140 also opens up the option of achieving synchronization of the delivery, in terms of time, of stimulation pulses delivered by the first stimulation unit 103 with stimulation pulses delivered by the second stimulation unit 120 via signals detected by the second detection unit 130.

A His bundle marker channel 150, a His bundle stimulation threshold test unit, which serves as a first stimulation threshold test device, and a His bundle diagnostic memory 190 are arranged downstream of the second timer 140.

It is possible, by way of the His bundle marker channel 150, to read out an electrocardiogram (ECG) or an intracardiac electrogram (IEGM) from the cardiac pacemaker 100, wherein the ECG or the IEGM is provided with markings that are specific to a His bundle stimulation by way of the second stimulation unit 120 and/or specific to the detection of a signal of the His bundle by way of the second detection unit 130. In this way, it is possible to retrieve ECGs or IEGMs provided with His bundle-specific information from the cardiac pacemaker 100 via the His bundle marker channel 150.

The His bundle stimulation threshold test unit 180 is used to determine a stimulus threshold of the His bundle before a corresponding stimulation of the His bundle is carried out by way of the second stimulation unit 120. In this way, it is possible, at all times, to provide a sufficiently strong stimulation pulse by way of the second stimulation unit 120, without having to expend more energy than necessary. The His bundle stimulation threshold test unit 180 is thus used, on the one hand, to ensure that the stimulation pulses delivered by the second stimulation unit 120 are strong enough to achieve an excitation of the His bundle, but, on the other hand, that the stimulation pulses delivered by the second stimulation unit 120 have the lowest possible energy, so that the load posed by the second stimulation unit 120 on the power source 101 of the cardiac pacemaker 100 is minimized.

Events that relate to the His bundle stimulation or the His bundle activity are stored in the His bundle diagnostic memory 190, which can be configured as a memory area of a larger memory unit, for example. The His bundle diagnostic memory is used, for example, to record stimulation pulses delivered by the second stimulation unit 120, and additionally to also record data detected by the second detection unit 130 with respect to the His bundle of the heart to be treated.

A His bundle remote monitoring and programming unit 200, which can be used to read out the His bundle diagnostic memory 190, is assigned to the His bundle diagnostic memory 190. In addition, the second stimulation unit 120 and/or the second detection unit 130 can be monitored by way of the His bundle remote monitoring and programming unit 200. It is further possible to adapt the His bundle stimulation to be delivered by the second stimulation unit 120 by way of the His bundle remote monitoring and programming unit 200. This His bundle remote monitoring and programming unit 200 thus allows access to specific components of the cardiac pacemaker 100, wherein it is made possible to both read out data and write data.

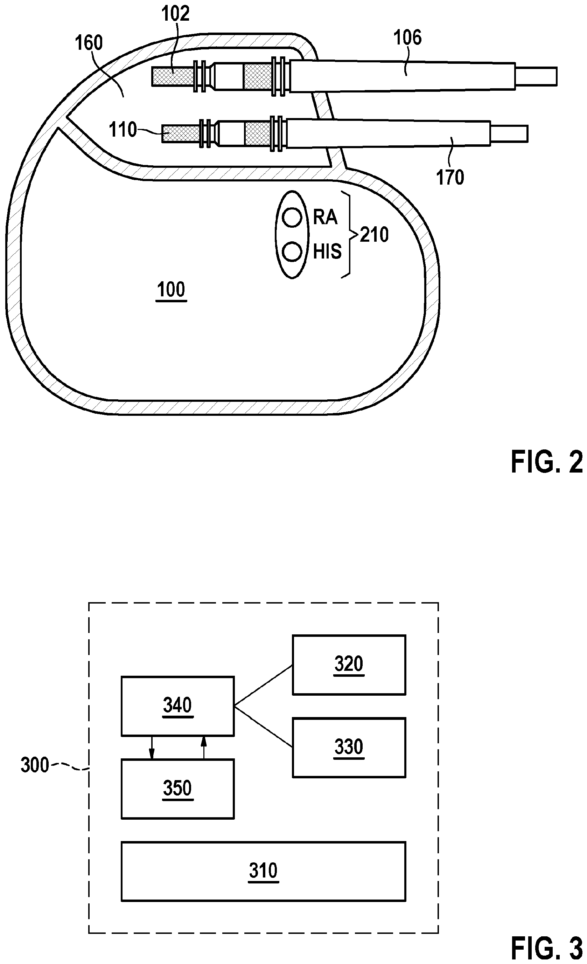

FIG. 2 shows a schematic side view of the cardiac pacemaker 100, of which the block diagram is shown in FIG. 1. Like elements are denoted by like reference numerals.

A header 160, in which the first electrode terminal 102 and the second electrode terminal 110 are formed, is apparent in the schematic illustration of FIG. 2. A first electrode 106 is plugged into the first electrode terminal 102, a second electrode 170 is plugged into the second electrode terminal 110. The first electrode 106 and the second electrode 107 are shown in sections in the illustration of FIG. 2.

The first electrode 106 is used to conventionally stimulate an arbitrary cardiac region of one of the ventricles. These are also used to detect electrical signals in one of these ventricles.

The second electrode 170 is used specifically to stimulate a His bundle and to detect His bundle-specific electrical signals. So as to enable easier use of the cardiac pacemaker 100 for a user, the second electrode 170 is identified as a His bundle electrode by the label "HIS."

To ensure that this second electrode 170 is plugged into the correct electrode terminal, this being the second electrode terminal 110, this terminal is provided with the additional identification "HIS" in the header 160 of the cardiac pacemaker 100.

It can also be provided that the second electrode terminal 110 is configured to be structurally different from the first electrode terminal 102, so that it is not possible to plug the second electrode 170 (this being the His bundle electrode) into the first electrode terminal 102 in the first place.

In addition to a label such as "HIS," color coding of the second electrode 170 and/or the second electrode terminal 110, or of a corresponding region of the header 160, can be provided.

On the side, the cardiac pacemaker 100 further includes a marking 210, which schematically represents the first electrode terminal 100 and the second electrode terminal 110 and provides it with a corresponding identification. The marking 210 indicates that the first (upper) electrode terminal 102 is provided for connecting a first electrode 106 leading into the right atrium (RA), and the second (lower) electrode terminal 110 is provided for connecting a second electrode 170 leading to the His bundle. This additional marking 210 additionally facilitates the connection of the correct electrodes 106, 170 to the intended electrode terminals 102, 110 for a user of the cardiac pacemaker 100.

FIG. 3 shows a block diagram of an exemplary embodiment of a cardiac pacemaker 300, which is used as an implantable system for stimulating the human or animal heart. This cardiac pacemaker 300 carries out stimulation outcome monitoring and is able to automatically adapt an internal control parameter as a function of the stimulation outcome monitoring that was carried out.

The cardiac pacemaker comprises a power source 310 and a His bundle stimulation unit 320, which serves as a stimulation unit. Furthermore, a detection unit 330 is provided, which serves as a detection unit. The His bundle stimulation unit 320 and the detection unit 330 are operatively connected to a processor 340. This processor 340, in turn, can access a memory unit 350 and receive data from or send data to this memory unit 350.

A program which the processor 340 can use to carry out certain steps is stored in the memory unit 350. For example, the processor 340 prompts the His bundle stimulation unit 320 to carry out a cardiac stimulation (in particular of the His bundle) by way of a stimulation electrode, which is not shown in FIG. 3. Thereafter, the processor 340 prompts the detection unit 330 to detect a cardiac electrical signal from the previously stimulated heart. This signal is then used to ascertain an excitation state of the heart.

The excitation state is classified into one of at least three different classes. Thereafter, a specific control parameter of the cardiac pacemaker 300 is automatically adapted as a function of the classification that was carried out.

The steps of ascertaining the excitation state, of classifying the excitation state, and of automatically adapting at least one control parameter can be carried out in a stimulation outcome monitoring module, which can be implemented either as hardware or as software. In the exemplary embodiment of FIG. 3, this stimulation outcome monitoring module is implemented as software, so that it is not shown separately.

As a result of the automatic adaptation of at least one control parameter of the cardiac pacemaker 300, a therapeutic outcome of a previously carried out His bundle stimulation can be monitored in a particularly simple manner, wherein a particularly safe and effective therapy can be ensured by the automatic adaptation of a control parameter, even over an extended period of time.

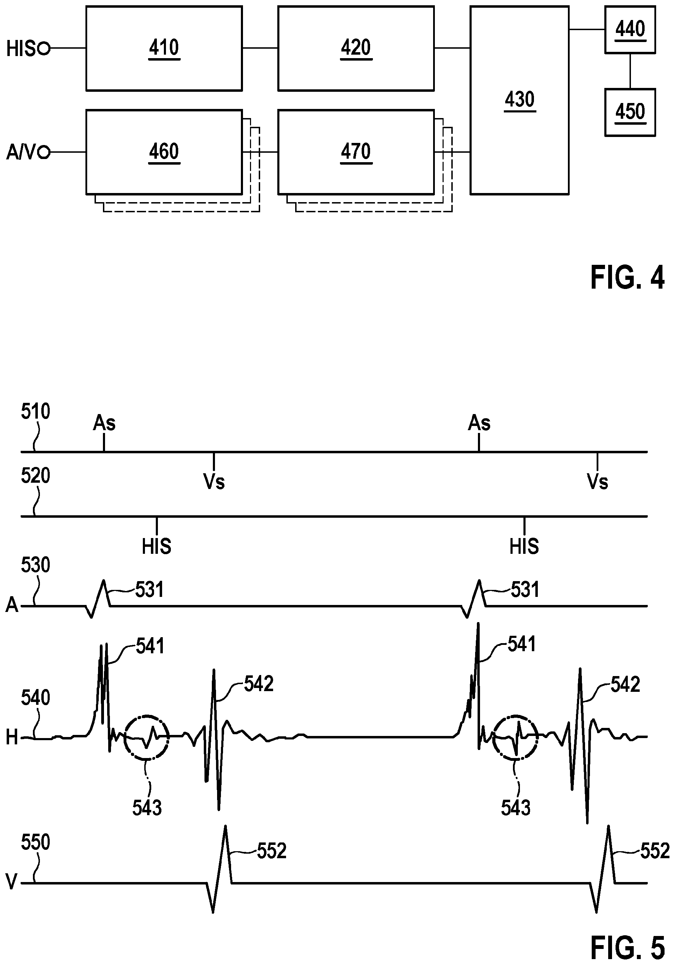

FIG. 4 shows a simplified block diagram of a His bundle pace-sense analyzer (His PSA), which serves as an analysis device. This His PSA comprises a His bundle stimulation unit 410, which includes an electrode interface by way of which a His bundle electrode can be connected to the His bundle stimulation unit 410. The His bundle stimulation unit 410 is used to deliver high energy stimulation, by which the His bundle of a human heart or an animal heart can be easily stimulated.

The His PSA further comprises a first detection unit 420, which can likewise directly access an electrode, by which a stimulation of the His bundle can be detected. The first detection unit 420 is provided and configured to record an electrocardiogram in the form of a broadband intracardiac electrogram (IEGM) and to subject it to a morphological signal analysis. Using a digital signal processor (DSP), which is an integral part of the first detection device 420, it is possible to identify and mark His bundle-specific signal morphologies within the captured electrocardiogram. The first detection unit 420 then forwards the further processed IEGM to a control and display unit 430, which serves as an output device for outputting the marked IEGM.

The control and display unit 430 is additionally connected to a processor 440, which, in turn, can access a memory unit 450. In this way, it is possible for the processor 440 to retrieve program information from the memory unit 450 and to transmit this information via the control and display unit 430 to the His bundle stimulation unit 410 and the first detection unit 420.

In addition, the His PSA comprises one or more further stimulation units 460, which are designed as conventional stimulation units and comprise conventional electrode interfaces for connecting atrial or ventricular electrodes. Conventional stimulation specifications of the corresponding electrodes can be provided in the process. In addition to the further stimulation units 460, one or more further detection units 470 are also provided, which are used to detect and evaluate atrial and ventricular cardiac signals. These further detection units 470 use conventional detection specifications for this purpose. Using these further detection units 470, it is possible to identify and mark ventricular and atrial signal in recorded electrocardiograms. The control and display unit 430 can thus display both His bundle-specific signals and atrial and/or ventricular signals in an electrocardiogram, such as an IEGM. The control and display unit 430 is able to represent different channels (in particular, an atrial channel, a ventricular channel, and a His bundle-specific channel) separately from one another.

This is shown schematically in FIG. 5 by way of example. For example, from top to bottom, FIG. 5 shows a first marker channel 510 for marking atrial signals As and ventricular signals Vs.

Beneath, a second marker channel 520 is shown, which is used to mark His bundle-specific signals HIS.

Only atrial signals 531 are schematically represented in an atrial IEGM channel 530.

In addition to atrial signals 541 and ventricular signals 542, His bundle-specific signals 543 are represented and separately marked in a His bundle-specific channel 540. This can typically be carried out by highlighting in color. In the illustration of FIG. 5, the His bundle-specific signals 543 are shown circled.

Finally, only ventricular signals 552 are schematically represented in a ventricular IEGM channel 550.

A user can have multiple channels 510 to 550 displayed simultaneously, or can have individual channels 510 to 550 displayed separately from other channels 510 to 550. In this way, it is possible to filter the information relevant for the particular problem for the user in a particularly simple manner. As a result of the automatic identification and separate marking of His bundle-specific signals 543, it is particularly easy to verify a correct positioning of a His bundle-specific stimulation electrode, and to optimize it with respect to the best possible contacting of the His bundle.

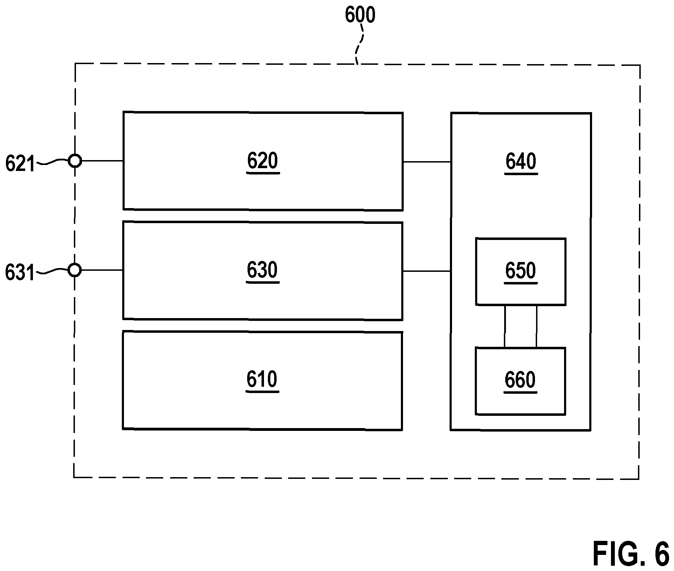

FIG. 6 shows a cardiac pacemaker 600, which is used as an implantable system for stimulating the human or animal heart. This cardiac pacemaker 600 is specifically provided and configured for treating an AV nodal reentry tachycardia (AVNRT).

The cardiac pacemaker 600 comprises a power source 610, a tachycardia identification and classification unit 620, which serves as a detection unit, and a His bundle stimulation unit 630, which serves as a stimulation unit. The tachycardia identification and classification unit 620 and the stimulation unit 630 are connected to a control unit 640. The control unit 640, in turn, is operatively connected to a processor 650, which can access a memory unit 660.

The tachycardia identification and classification unit 620 comprises an electrode terminal 621 to which a first electrode can be connected. By way of this first electrode, the tachycardia identification and classification unit 620 is able to identify whether tachycardia is present in the heart of the patient wearing the cardiac pacemaker 600. The tachycardia identification and classification unit 620 is additionally able to identify and classify the type of tachycardia. In particular, an AV nodal reentry tachycardia can be distinguished by the tachycardia identification and classification unit 620 from other tachycardias.

When such an AV nodal reentry tachycardia is identified by the tachycardia identification and classification unit 620, the control unit 640 ensures that the stimulation unit 630 delivers a stimulation that is specifically suitable for stimulating the His bundle of the heart of the patient. For this purpose, the stimulation unit 630 comprises an electrode terminal 631, to which a second electrode, which is arranged in the his bundle or so close to the His bundle that a His bundle stimulation is possible by way of this electrode, is connected during operation of the cardiac pacemaker 600. The control unit 640 transmits the corresponding signal to the stimulation unit 630 after having received a corresponding command from the processor 650. A program which the processor 650 obtains from the memory unit 660 runs on the processor 650.

The individual components of the cardiac pacemaker 600 are supplied with the power necessary for operation from the power source 610.

The tachycardia identification and classification unit 620 uses ECG signals, received via the electrode terminal 621, for identifying and classifying a tachycardia, and in particular for identifying an AV nodal reentry tachycardia. The ECG signals can be derived from the atrium and/or the ventricle of the heart of the patient. In addition, it is possible to provide corresponding ECG signals directly via a His bundle electrode, for example via the second electrode , which is also connected to the second electrode terminal 631 of the stimulation unit 630.

So as to classify the established tachycardia, that is, so as to distinguish different tachycardias from one another, and, in particular, so as to identify an AV nodal reentry tachycardia, the tachycardia identification and classification unit 620 can resort to morphological analysis criteria within the provided electrocardiogram or, instead of such a morphological analysis of the electrocardiogram, can evaluate a chronological sequence of atrial and/or ventricular signals in the electrocardiogram. It is also possible to carry out a morphological analysis of a provided electrocardiogram, and to carry out an analysis of the chronological sequence of the signals present in this electrocardiogram.

This cardiac pacemaker 600 provides a new, device-based therapy for tachycardias, and in particular, for AV node reentry tachycardia. As a result, the treatment spectrum of active implants is enhanced.

## Claims

1. An implantable system for stimulating a human heart or an animal heart, comprising a first stimulation unit (103) and a first detection unit (104), the first stimulation unit (103) being used to stimulate at least one cardiac region of a human heart or an animal heart, and the first detection unit (104) being used to detect an electrical signal of at least one cardiac region of the same human or animal heart, the system comprises a second stimulation unit (120), which is specifically designed and configured to stimulate a His bundle of the same human or animal heart,
**characterized in that**
the system comprises a first timer (105), which is used to provide a defined delivery of stimulation pulses, in terms of time, by the first stimulation unit (103), and the system comprises a second timer (140), which is provided and configured to match a delivery point in time of at least one pulse to be delivered by the second stimulation unit (120) to a delivery point in time of at least one pulse to be delivered by the first stimulation unit (103).

2. The implantable system according to claim 1, **characterized in that** the system comprises a second detection unit (130), which is specifically designed and configured to detect an electrical signal of the His bundle of the same human or animal heart, the second detection unit (130) for the specific design and configuration thereof having at least one of the following features: a) a sensitivity that is at least 10% higher than the sensitivity of the first detection unit (104); b) a detection range that is at least 10% greater than the detection range of the first detection unit (104); and c) a sampling rate that is at least 10% higher than the sampling rate of the first detection unit (104).

3. The implantable system according to claim 1 or 2, **characterized in that** the second stimulation unit (120) comprises a maximum stimulation energy that is at least 10% higher than the maximum stimulation energy of the first stimulation unit (103).

4. The implantable system according to any of the preceding claims, **characterized in that** the second timer (140) is also used to match a delivery point in time of at least one pulse to be delivered by the first stimulation unit (103) to a delivery point in time of at least one pulse to be delivered by the second stimulation unit (120).

5. The implantable system according to any one of the preceding claims, **characterized in that** the system comprises a first stimulation threshold test device (180), by which a stimulus threshold of the His bundle of a human heart or an animal heart to be stimulated by the device can be ascertained, a stimulation energy of the second stimulation unit (120) being adjustable as a function of the ascertained stimulus threshold.

6. The implantable system according to any one of claims 2 to 5, **characterized in that** the system comprises a memory unit, which includes a memory area (190) that is used exclusively for storing data collected by the second detection unit (130) and/or data related to a stimulation delivered by the second stimulation unit (120).

7. The implantable system according to any one of claims 2 to 6, **characterized in that** the system comprises a remote communication unit (200), which is used to transmit data collected by the second detection unit (130), and/or data related to an activity of the second stimulation unit (120), and/or to monitor the second detection unit (130) and/or the second stimulation unit (120), and/or to adapt a stimulation to be delivered by the second stimulation unit (120).

8. The implantable system according to any one of claims 2 to 7, **characterized in that** the system comprises a processor and a memory unit, the memory unit including a computer-readable program that prompts the processor to carry out the following steps when the program is being executed on the processor: a) transferring the system into a His bundle stimulation mode, in which a His bundle stimulation can only be carried out by way of the second stimulation unit (120); and b) delivering a His bundle stimulation by way of the second stimulation unit (120) when an event warranting the delivery was detected by way of the second detection unit (130).

9. The implantable system according to any one of claims 2 to 8, **characterized in that** the system comprises a processor and a memory unit, the memory unit including a computer-readable program that prompts the processor to carry out the following steps when the program is being executed on the processor: a) transferring the system into a safety mode, in which at least one stimulation of at least one ventricle can be carried out by way of the first stimulation unit (103) or a His bundle stimulation can be carried out by way of the second stimulation unit (120); and b) delivering a ventricular stimulation by way of the first stimulation unit (103) and/or delivering a His bundle stimulation by way of the second stimulation unit (120) when an event warranting the delivery was detected by way of the first detection unit (104) and/or the second detection unit (130).

10. The implantable system according to any one of claims 2 to 9, **characterized in that** the system comprises a marker channel (150), which can be used to read out an electrocardiogram (ECG) or an intracardiac electrogram (IEGM), the electrocardiogram or the intracardiac electrogram including at least one marking specific to a His bundle stimulation by way of the second stimulation unit (120) and/or specific to a detection of a signal of the His bundle by way of the second detection unit (130).

11. A computer program product including computer-readable code, which prompts a processor to carry out the following steps when the code is being executed on the processor.
a) detecting by way of a first detection unit (104) and/or a second detection unit (130) whether a cardiac rhythm disturbance to be treated is present in a human heart or an animal heart, the first detection unit (104) being provided to detect an electrical signal of at least one cardiac region of the human or animal heart, and the second detection unit (130) being specifically designed and configured to detect an electrical signal of the His bundle of the same human or animal heart; and
b) carrying out an atrial or a ventricular stimulation by way of a first stimulation unit (103) and/or carrying out a His bundle stimulation by way of a second stimulation unit (120) when a cardiac rhythm disturbance to be treated is present,
wherein a first timer (105) is used to provide a defined delivery of stimulation pulses, in terms of time, by the first stimulation unit (103), and a second timer (140) is used to match a delivery point in time of at least one pulse to be delivered by the second stimulation unit (120) to a delivery point in time of at least one pulse to be delivered by the first stimulation unit (103).

## Patentansprüche

1. Implantierbares System für eine Stimulierung eines Herzens eines Menschen oder eines Tiers, eine erste Stimulationseinheit (103) und eine erste Detektionseinheit (104) umfassend, wobei die erste Stimulationseinheit (103) verwendet wird, um mindestens eine kardiale Region eines Herzens eines Menschen oder eines Tiers zu stimulieren, und die erste Detektionseinheit (104) verwendet wird, um ein elektrisches Signal mindestens einer kardialen Region dieses Herzens eines Menschen oder eines Tiers zu detektieren, wobei das System eine zweite Stimulationseinheit (120) umfasst, die spezifisch dafür ausgelegt und konfiguriert ist, ein His-Bündel dieses Herzens eines Menschen oder eines Tiers zu stimulieren,
**dadurch gekennzeichnet, dass**
das System einen ersten Zeitgeber (105) umfasst, der verwendet wird, um eine hinsichtlich der Zeit definierte Abgabe von Stimulationsimpulsen durch die erste Stimulationseinheit (103) bereitzustellen, und das System einen zweiten Zeitgeber (140) umfasst, der bereitgestellt und konfiguriert ist, um einen Abgabezeitpunkt mindestens eines von der zweiten Stimulationseinheit (120) abzugebenden Impulses mit einem Abgabezeitpunkt mindestens eines von der ersten Stimulationseinheit (103) abzugebenden Impulses in Übereinstimmung zu bringen.

2. Implantierbares System nach Anspruch 1, **dadurch gekennzeichnet, dass** das System eine zweite Detektionseinheit (130) umfasst, die spezifisch dafür ausgelegt und konfiguriert ist, ein elektrisches Signal des His-Bündels dieses Herzens eines Menschen oder eines Tiers zu detektieren, wobei die zweite Detektionseinheit (130) für ihr spezifisches Design und ihre spezifische Konfiguration mindestens eines der folgenden Merkmale aufweist: a) eine Empfindlichkeit, die um mindestens 10 % höher ist als die Empfindlichkeit der ersten Detektionseinheit (104); b) einen Detektionsbereich, der um mindestens 10 % größer ist als der Detektionsbereich der ersten Detektionseinheit (104); und c) eine Abtastrate, die um mindestens 10 % höher ist als die Abtastrate der ersten Detektionseinheit (104).

3. Implantierbares System nach Anspruch 1 oder 2, **dadurch gekennzeichnet, dass** die zweite Stimulationseinheit (120) eine maximale Stimulationsenergie umfasst, die um mindestens 10 % höher ist als die maximale Stimulationsenergie der ersten Stimulationseinheit (103).

4. Implantierbares System nach einem der vorangehenden Ansprüche, **dadurch gekennzeichnet, dass** der zweite Zeitgeber (140) auch verwendet wird, um einen Abgabezeitpunkt mindestens eines von der ersten Stimulationseinheit (103) abzugebenden Impulses mit einem Abgabezeitpunkt mindestens eines von der zweiten Stimulationseinheit (120) abzugebenden Impulses in Übereinstimmung zu bringen.

5. Implantierbares System nach einem der vorangehenden Ansprüche, **dadurch gekennzeichnet, dass** das System eine erste Stimulationsschwellenwert-Testvorrichtung (180) umfasst, durch die ein Stimulationsschwellenwert des His-Bündels eines von der Vorrichtung zu stimulierenden Herzens eines Menschen oder eines Tiers festgestellt werden kann, wobei eine Stimulationsenergie der zweiten Stimulationseinheit (120) als Funktion des festgestellten Stimulationsschwellenwerts anpassbar ist.

6. Implantierbares System nach einem der Ansprüche 2 bis 5, **dadurch gekennzeichnet, dass** das System eine Speichereinheit umfasst, die einen Speicherbereich (190) aufweist, der ausschließlich zum Speichern von Daten, die von der zweiten Detektionseinheit (130) erfasst werden, und/oder von Daten, die eine von der zweiten Stimulationseinheit (120) abgegebene Stimulation betreffen, verwendet wird.

7. Implantierbares System nach einem der Ansprüche 2 bis 6, **dadurch gekennzeichnet, dass** das System eine Fernkommunikationseinheit (200) umfasst, die verwendet wird, um Daten, die von der zweiten Detektionseinheit (130) erfasst werden, und/oder Daten, die eine Aktivität der zweiten Stimulationseinheit (120) betreffen, zu senden und/oder die zweite Detektionseinheit (130) und/oder die zweite Stimulationseinheit (120) zu überwachen und/oder eine von der zweiten Stimulationseinheit (120) abzugebende Stimulation anzupassen.

8. Implantierbares System nach einem der Ansprüche 2 bis 7, **dadurch gekennzeichnet, dass** das System einen Prozessor und eine Speichereinheit umfasst, wobei die Speichereinheit ein computerlesbares Programm aufweist, das den Prozessor veranlasst, die folgenden Schritte auszuführen, wenn das Programm auf dem Prozessor ausgeführt wird: a) Überführen des Systems in einen His-Bündel-Stimulationsmodus, in dem eine His-Bündel-Stimulation nur mittels der zweiten Stimulationseinheit (120) ausgeführt werden kann; und b) Abgeben einer His-Bündel-Stimulation mittels der zweiten Stimulationseinheit (120), wenn mittels der zweiten Detektionseinheit (130) ein Ereignis detektiert wurde, das die Abgabe rechtfertigt.

9. Implantierbares System nach einem der Ansprüche 2 bis 8, **dadurch gekennzeichnet, dass** das System einen Prozessor und eine Speichereinheit umfasst, wobei die Speichereinheit ein computerlesbares Programm aufweist, das den Prozessor veranlasst, die folgenden Schritte auszuführen, wenn das Programm auf dem Prozessor ausgeführt wird: a) Überführen des Systems in einen Sicherheitsmodus, in dem mindestens eine Stimulation mindestens eines Ventrikels mittels der ersten Stimulationseinheit (103) ausgeführt werden kann oder eine His-Bündel-Stimulation mittels der zweiten Stimulationseinheit (120) ausgeführt werden kann; und b) Abgeben einer ventrikulären Stimulation mittels der ersten Stimulationseinheit (103) und/oder Abgeben einer His-Bündel-Stimulation mittels der zweiten Stimulationseinheit (120), wenn mittels der ersten Detektionseinheit (104) und/oder der zweiten Detektionseinheit (130) ein Ereignis detektiert wurde, das die Abgabe rechtfertigt.

10. Implantierbares System nach einem der Ansprüche 2 bis 9, **dadurch gekennzeichnet, dass** das System einen Markerkanal (150) umfasst, der verwendet werden kann, um ein Elektrokardiogramm (EKG) oder ein intrakardiales Elektrogramm (IEGM) auszulesen, wobei das intrakardiale Elektrogramm mindestens eine Markierung aufweist, die für eine His-Bündel-Stimulation anhand der zweiten Stimulationseinheit (120) spezifisch ist und/oder die für eine Detektion eines Signals des His-Bündels anhand der zweiten Detektionseinheit (130) spezifisch ist.

11. Computerprogrammprodukt, einen computerlesbaren Code aufweisend, der einen Prozessor veranlasst, die folgenden Schritte auszuführen, während der Code auf dem Prozessor ausgeführt wird:
a) Detektieren, ob eine behandlungsbedürftige Herzrhythmusstörung in einem Herzen eines Menschen oder eines Tiers vorliegt, anhand einer ersten Detektionseinheit (104) und/oder einer zweiten Detektionseinheit (130), wobei die erste Detektionseinheit (104) bereitgestellt ist, um ein elektrisches Signal mindestens einer kardialen Region des Herzens eines Menschen oder eines Tiers zu detektieren, und die zweite Detektionseinheit (130) spezifisch dafür ausgelegt und konfiguriert ist, ein elektrisches Signal des His-Bündels dieses Herzens eines Menschen oder eines Tiers zu detektieren; und
b) Ausführen einer atrialen oder einer ventrikulären Stimulation anhand einer ersten Stimulationseinheit (103) und/oder Ausführen einer His-Bündel-Stimulation anhand einer zweiten Stimulationseinheit (120), wenn eine behandlungsbedürftige Herzrhythmusstörung vorliegt,
wobei ein erster Zeitgeber (105) verwendet wird, um eine hinsichtlich der Zeit definierte Abgabe von Stimulationsimpulsen durch die erste Stimulationseinheit (103) bereitzustellen, und ein zweiter Zeitgeber (140) verwendet wird, um einen Abgabezeitpunkt mindestens eines von der zweiten Stimulationseinheit (120) abzugebenden Impulses mit einem Abgabezeitpunkt mindestens eines von der ersten Stimulationseinheit (103) abzugebenden Impulses in Übereinstimmung zu bringen.

## Revendications

1. Système implantable destiné à la stimulation d'un coeur humain ou d'un coeur animal, comprenant une première unité de stimulation (103) et une première unité de détection (104), la première unité de stimulation (103) étant utilisée pour stimuler au moins une région cardiaque d'un coeur humain ou d'un coeur animal, et la première unité de détection (104) étant utilisée pour détecter un signal électrique d'au moins une région cardiaque du même coeur humain ou animal, le système comprenant une seconde unité de stimulation (120), qui est spécifiquement conçue et configurée pour stimuler un faisceau de His du même coeur humain ou animal,
**caractérisé en ce que**
le système comprend un premier chronomètre (105), qui est utilisé pour fournir une administration définie d'impulsions de stimulation, en termes de temps, par la première unité de stimulation (103), et le système comprend un second chronomètre (140), qui est fourni et configuré pour faire correspondre un point d'administration dans le temps d'au moins une impulsion à administrer par la seconde unité de stimulation (120) à un point d'administration dans le temps d'au moins une impulsion à administrer par la première unité de stimulation (103).

2. Système implantable selon la revendication 1, **caractérisé en ce que** le système comprend une seconde unité de détection (130), qui est spécifiquement conçue et configurée pour détecter un signal électrique du faisceau de His du même coeur humain ou animal, la seconde unité de détection (130) pour la conception et la configuration spécifiques de celle-ci ayant au moins l'une des caractéristiques suivantes : a) une sensibilité supérieure d'au moins 10 % à la sensibilité de la première unité de détection (104) ; b) une plage de détection qui est supérieure d'au moins 10 % à la plage de détection de la première unité de détection (104) ; et c) un taux d'échantillonnage qui est supérieur d'au moins 10 % au taux d'échantillonnage de la première unité de détection (104).

3. Système implantable selon la revendication 1 ou 2, **caractérisé en ce que** la seconde unité de stimulation (120) comprend une énergie de stimulation maximale qui est supérieure d'au moins 10 % à l'énergie de stimulation maximale de la première unité de stimulation (103).

4. Système implantable selon l'une quelconque des revendications précédentes, **caractérisé en ce que** le second chronomètre (140) est également utilisé pour faire correspondre un point d'administration dans le temps d'au moins une impulsion à administrer par la première unité de stimulation (103) à un point d'administration dans le temps d'au moins une impulsion à administrer par la seconde unité de stimulation (120).

5. Système implantable selon l'une quelconque des revendications précédentes, **caractérisé en ce que** le système comprend un premier dispositif de test de seuil de stimulation (180), par lequel un seuil de stimulus du faisceau de His d'un coeur humain ou d'un coeur animal à stimuler par le dispositif peut être déterminé, une énergie de stimulation de la seconde unité de stimulation (120) étant ajustable en fonction du seuil de stimulus déterminé.

6. Système implantable selon l'une quelconque des revendications 2 à 5, **caractérisé en ce que** le système comprend une unité de mémoire, qui inclut une zone de mémoire (190) qui est utilisée exclusivement pour le stockage de données recueillies par la seconde unité de détection (130) et/ou de données liées à une stimulation administrée par la seconde unité de stimulation (120).

7. Système implantable selon l'une quelconque des revendications 2 à 6, **caractérisé en ce que** le système comprend une unité de communication à distance (200), qui est utilisée pour transmettre des données recueillies par la seconde unité de détection (130), et/ou des données liées à une activité de la seconde unité de stimulation (120), et/ou pour surveiller la seconde unité de détection (130) et/ou la seconde unité de simulation (120), et/ou pour adapter une stimulation à administrer par la seconde unité de stimulation (120).

8. Système implantable selon l'une quelconque des revendications 2 à 7, **caractérisé en ce que** le système comprend un processeur et une unité de mémoire, l'unité de mémoire incluant un processeur lisible par ordinateur qui invite le processeur à mettre en oeuvre les étapes suivantes lorsque le programme est exécuté sur le processeur : a) transfert du système dans un mode de stimulation de faisceau de His, dans lequel une stimulation de faisceau de His peut uniquement être mise en oeuvre par le biais de la seconde unité de stimulation (120) ; et b) administration d'une stimulation de faisceau de His par le biais de la seconde unité de stimulation (120) lorsqu'un événement nécessitant l'administration a été détecté par le biais de la seconde unité de détection (130).

9. Système implantable selon l'une quelconque des revendications 2 à 8, **caractérisé en ce que** le système comprend un processeur et une unité de mémoire, l'unité de mémoire incluant un processeur lisible par ordinateur qui invite le processeur à mettre en oeuvre les étapes suivantes lorsque le programme est exécuté sur le processeur : a) transfert du système dans un mode de sécurité, dans lequel au moins une stimulation d'au moins un ventricule peut être mise en oeuvre par le biais de la première unité de stimulation (103) ou une stimulation de faisceau de His peut être mise en oeuvre par le biais de la seconde unité de stimulation (120) ; et b) administration d'une stimulation ventriculaire par le biais de la première unité de stimulation (103) et/ou administration d'une stimulation de faisceau de His par le biais de la seconde unité de stimulation (120) lorsqu'un événement nécessitant l'administration a été détecté par le biais de la première unité de détection (104) et/ou de la seconde unité de détection (130).

10. Système implantable selon l'une quelconque des revendications 2 à 9, **caractérisé en ce que** le système comprend un canal marqueur (150), qui peut être utilisé pour lire un électrocardiogramme (ECG) ou un électrogramme intracardiaque (IEGM), l'électrocardiogramme ou l'électrogramme intracardiaque incluant au moins une marque spécifique d'une stimulation de faisceau de His par le biais de la seconde unité de stimulation (120) et/ou spécifique d'une détection d'un signal du faisceau de His par le biais de la seconde unité de détection (130).

11. Produit de programme informatique incluant un code lisible par ordinateur, qui invite un processeur à mettre en oeuvre les étapes suivantes lorsque le code est exécuté sur le processeur :
a) détection par le biais d'une première unité de détection (104) et/ou d'une seconde unité de détection (130) si une perturbation du rythme cardiaque à traiter est présente dans un coeur humain ou un coeur animal, la première unité de détection (104) étant fournie pour détecter un signal électrique d'au moins une région cardiaque du coeur humain ou animal, et la seconde unité de détection (130) étant spécifiquement conçue et configurée pour détecter un signal électrique du faisceau de His du même coeur humain ou animal ; et
b) mise en oeuvre d'une stimulation auriculaire ou ventriculaire par le biais d'une première unité de stimulation (103) et/ou mise en oeuvre d'une stimulation de faisceau de His par le biais d'une seconde unité de stimulation (120) lorsqu'une perturbation de rythme cardiaque à traiter est présente,
dans lequel un premier chronomètre (105) est utilisé pour fournir une administration définie d'impulsions de stimulation, en termes de temps, par la première unité de stimulation (103), et un second chronomètre (140) est utilisé pour faire correspondre un point d'administration dans le temps d'au moins une impulsion à administrer par la seconde unité de stimulation (120) à un point d'administration dans le temps d'au moins une impulsion à administrer par la première unité de stimulation (103).
